# EUROPEAN PATENT APPLICATION

(11) **EP 4 667 934 A1**
(43) Date of publication of application: **24.12.2025**
(21) Application number: 25177886.6
(22) Date of filing: 21.05.2025
(51) Int. Cl.: G01N 33/24

(54) **METHOD FOR SEPARATING AND ANALYZING BIOMARKERS FROM SEDIMENTS**

(30) Priority: 19.06.2024 KR 20240079931
(71) Applicant: KOREA INSTITUTE OF GEOSCIENCE AND MINERAL RESOURCES, Yuseong-gu Daejeon 34132 (KR)
(72) Inventor: Choi, Jiyoung, 34066 Daejeon (KR)
(74) Representative: Witte, Weller & Partner Patentanwälte mbB

(57) **Abstract**

The present disclosure provides a method for separating and analyzing biomarkers from sediments, source rocks, or reservoir rocks. Conventional techniques require different separation and analysis procedures depending on the type of biomarker, leading to increased process complexity and cost. To address these limitations, the present disclosure enables the simultaneous separation and analysis of various types of biomarkers through a single process, thereby improving efficiency and reducing operational costs.

## Description

### BACKGROUND OF THE DISCLOSURE

### 1. Field of the Disclosure

The present disclosure relates to a method for separating and analyzing geological biomarkers. In particular, the present disclosure relates to a method for separating and analyzing biomarkers from sediments, source rocks, or reservoir rocks.

### 2. Description of the Related Art

Geological biomarkers were first defined by Eglinton and Calvin (1967). In general, geological biomarkers function like a kind of fingerprint found in sediments and are actively used in studies on the origin of sediments, depositional environments, maturity, and biodegradation processes.

The reason why geological biomarkers can serve as such a powerful material (tool) in research is quite simple. Sediments contain organic matter, which is defined as compounds composed of carbon linkages. The organic matter in sediments, which consists of carbon-based compounds, originates from organisms or plants that existed at the time of deposition.

For example, steranes are modified forms of sterols, and porphyrins are altered forms of chlorophyll that have undergone transformation after deposition. Therefore, by studying organic compounds present in sediments (in particular, by studying biomarkers), it is possible to investigate not only the origin of the organic matter but also the environmental processes it underwent after deposition.

This organic matter can be broadly classified into insoluble organic matter and soluble organic matter. Kerogen belongs to the category of insoluble organic matter. Among these, the substances referred to as biomarkers are found in the soluble organic matter. Soluble organic matter is further categorized into aliphatic hydrocarbons, aromatic hydrocarbons, and NSO compounds, depending on the types of compounds and the bonding forms of carbon.

Conventional methods for separating and analyzing biomarkers from sediments, source rocks, or reservoir rocks have the inconvenience of requiring different separation procedures depending on the type of biomarker to be isolated, depending on whether it is an aliphatic hydrocarbon (particularly saturated hydrocarbons), an aromatic hydrocarbon, or an NSO compound.

Accordingly, the inventors of the present disclosure have developed a method capable of simultaneously separating and analyzing various types of biomarkers from sediments, source rocks, or reservoir rocks through a single process, and have confirmed its effectiveness, thereby completing the present disclosure.

### Related Patent Document

Korean Registered Patent No. 10-1694994 (January 11, 2017)

### SUMMARY OF THE DISCLOSURE

The purpose of the present disclosure is to provide a method for simultaneously separating and analyzing various types of biomarkers from sediments, source rocks, or reservoir rocks through a single process.

The challenges that the present dislosure is intended to solve are not limited to those mentioned above, and other challenges not mentioned will be apparent to those skilled in the art from the following description.

In order to achieve the purpose, an aspect of the present disclosure provides a method for separating and analyzing biomarkers, comprising:
(a) extracting organic matter from an original sample;
(b) removing inorganic matter from a sample obtained in step (a);
(c) removing asphaltene from a sample obtained in step (b);
(d) classifying a sample obtained in step (c) into a group containing saturated hydrocarbons, a group containing aromatic hydrocarbons, and a group containing NSO compounds; and
(e) detecting components included in each of the group containing saturated hydrocarbons, the group containing aromatic hydrocarbons, and the group containing NSO compounds, all of which are obtained in step (d).

In some exemplary embodiments, the sample in step (a) may include a mixture of one or more selected from the group consisting of soil, rock, sediment, marine sediment, sedimentary rock, source rock, and reservoir rock.

In some exemplary embodiments, step (a) may include grinding the original sample prior to the extraction of organic matter.

In some exemplary embodiments, step (a) may include performing distillation with an organic solvent on a sample including rock, sediment, or a mixture thereof, and the sample does not contain oil or bitumen.

In some exemplary embodiments, step (a) may include performing ultrasonic crushing after adding an organic solvent to a sample containing oil, bitumen, or a mixture thereof.

In some exemplary embodiments, step (b) may be performed using filtration.

In some exemplary embodiments, step (c) may be performed by mixing a non-polar organic solvent with the sample obtained in step (b).

In some exemplary embodiments, step (d) may be performed by sequentially separating:
a group containing saturated hydrocarbons using a first solvent,
a group containing aromatic hydrocarbons using a second solvent, and
a group containing NSO compounds using a third solvent.

In some exemplary embodiments, the first solvent may be an organic solvent having a polarity index of 0 to 0.5, the second solvent may be an organic solvent having a polarity index of 1 to 3, and the third solvent may be an organic solvent having a polarity index of 4 to 5.

In some exemplary embodiments, step (e) may be performed using GC or GC/MS.

According to the present disclosure, various types of biomarkers can be simultaneously separated and analyzed from sediments, source rocks, or reservoir rocks through a single process.

The effects of the present disclosure are not limited to the aforementioned effects and should be understood to include all effects that can be inferred from the configurations of the present disclosure described in the detailed description or the claims.

### BRIEF DESCRIPTION OF THE DRAWINGS

FIG. 1 is a process flow diagram of a method for separating and analyzing biomarkers according to an exemplary embodiment of the present disclosure.
FIG. 2 is a photograph showing the agate-mortar cleaning method and sample grinding used in the sample preparation step according to an exemplary embodiment of the present disclosure.
FIG. 3 is a graph for determining the weight of the sample to be used for extraction in the sample preparation step according to an exemplary embodiment of the present disclosure (X-axis: EOM content, Y-axis: Sample weight × S1).
FIG. 4 is a photograph showing the Soxhlet apparatus used in the extraction step for organic matter from rock and sediment samples according to an exemplary embodiment of the present disclosure.
FIG. 5 is a photograph showing copper wire stored in a MeOH solution and 10 % hydrochloric acid used to remove free sulfate from samples in the extraction step of organic matter from rock and sediment samples according to an exemplary embodiment of the present disclosure.
FIG. 6 is a photograph showing a sonicator and a centrifuge used in the extraction step of organic matter from oil and bitumen samples according to an exemplary embodiment of the present disclosure.
FIG. 7 is a photograph showing a filter using glass wool and an evaporator used in the inorganic matter removal step according to an exemplary embodiment of the present disclosure.
FIG. 8 is a photograph showing solvents before and after filtration in the inorganic matter removal step according to an exemplary embodiment of the present disclosure.
FIG. 9 is a photograph showing asphaltene precipitation and coagulation after mixing pentane with a sample in the asphaltene removal step according to an exemplary embodiment of the present disclosure.
FIG. 10 is a photograph showing the elution of sample components by group in the biomarker classification step according to an exemplary embodiment of the present disclosure.
FIG. 11 is a photograph showing the GC/MS instrument used according to an exemplary embodiment of the present disclosure.
FIG. 12 shows GC/MS results from an m/z 191 fragmentogram according to an exemplary embodiment of the present disclosure.
FIG. 13 shows the chemical structures of Hopane and 25norHopane according to an exemplary embodiment of the present disclosure.
FIG. 14 shows GC/MS results from an m/z 177 fragmentogram according to an exemplary embodiment of the present disclosure.
FIG. 15 shows GC/MS results from an m/z 217 fragmentogram according to an exemplary embodiment of the present disclosure.
FIG. 16 shows the chemical structures of various Naphthalenes according to an exemplary embodiment of the present disclosure.
FIG. 17 shows GC/MS results from m/z 128, 142, 156, 170, and 184 fragmentograms according to an exemplary embodiment of the present disclosure.
FIG. 18 shows the chemical structures of various Phenanthrenes according to an exemplary embodiment of the present disclosure.
FIG. 19 shows GC/MS results from m/z 178, 192, and 206 fragmentograms according to an exemplary embodiment of the present disclosure.
FIG. 20 shows the chemical structures of various Benzothiophenes according to an exemplary embodiment of the present disclosure.
FIG. 21 shows GC/MS results from m/z 184, 198, and 212 fragmentograms according to an exemplary embodiment of the present disclosure.

### DETAILED DESCRIPTION OF THE DISCLOSURE

In the following description, only the parts necessary for understanding an exemplary embodiment of the present disclosure are described, and it should be noted that other parts are omitted to the extent that such omission does not detract from the gist of the present disclosure.

The terms and words used in the specification and claims below should not be interpreted as being limited to their conventional or dictionary definitions, but should be construed in accordance with the meanings and concepts consistent with the technical spirit of the present disclosure, based on the principle that the inventor may appropriately define the terms to best explain the invention.

Accordingly, the exemplary embodiments described in this specification and the configurations illustrated in the drawings are merely exemplary and do not represent the entirety of the technical spirit of the present disclosure. It should therefore be understood that various equivalents and modifications capable of substituting them may exist at the time of filing.

Hereinafter, exemplary embodiments of the present disclosure will be described in detail.

The present disclosure provides a method for separating and analyzing biomarkers, comprising: (a) extracting organic matter from an original sample; (b) removing inorganic matter from a sample obtained in step (a); (c) removing asphaltene from a sample obtained in step (b); (d) classifying a sample obtained in step (c) into a group containing saturated hydrocarbons, a group containing aromatic hydrocarbons, and a group containing NSO compounds; and (e) detecting components included in each of the group containing saturated hydrocarbons, the group containing aromatic hydrocarbons, and the group containing NSO compounds, all of which are obtained in step (d).

For clarity, it is to be understood that the phrase "each of the group containing..." as used above is intended to refer to each of the three groups described in step (d), namely, the group containing saturated hydrocarbons, the group containing aromatic hydrocarbons, and the group containing NSO compounds.

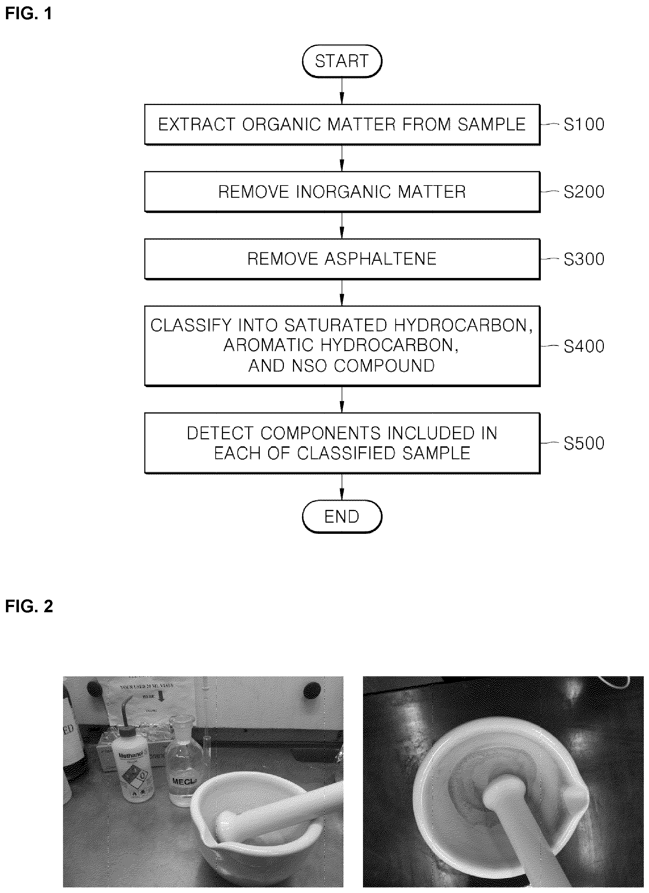

Hereinafter, the description will be made with reference to FIG. 1.

Step (a): Extracting organic matter from an original sample (S100).

The original sample may be soil or rock, and more specifically, may comprise one or more mixtures selected from sediment, marine sediment, sedimentary rock or sedimentary structure formed therefrom, source rock, or reservoir rock.

This step may further include grinding the original sample prior to the extraction of organic matter.

In an exemplary embodiment, the original sample may have an average particle size of 100 mesh or less for oil or bitumen-containing samples.

In respect to the sample containing rock, sediment, or a mixture thereof, which does not contain oil or bitumen, organic matter may be extracted by performing distillation with an organic solvent. Specifically, the extraction may be carried out using a Soxhlet apparatus. In this case, the step may further include removing free sulfate present in the sample.

In respect to the sample containing oil, bitumen, or a mixture thereof, organic matter may be extracted by adding an organic solvent followed by ultrasonic crushing. Afterwards, the extraction may further include centrifugation.

The organic solvent used for both types of samples may have a polarity index of 3 to 5, and preferably 3.5 to 4.5. In particular, the organic solvent may be a mixture of DCM:MeOH in a volume ratio of 3:7 to 7:3 (v/v), 4:6 to 6:4 (v/v), or 1:1 (v/v).

Step (b): Removing inorganic matter from the sample obtained in step S100 (S200).

This step may involve removing inorganic matter, particularly clay minerals, from the solvent containing the organic matter obtained in step S100 by filtration. In particular, the filtration may be performed using glass wool.

In addition, this step may further include distilling the filtered solvent as described above.

Step (c): Removing asphaltenes from the sample obtained in step S200 (S300).

Asphaltenes are highly polar and high-molecular-weight substances that significantly interfere with biomarker analysis. Therefore, it is necessary to effectively remove them.

Therefore, in this step, asphaltenes are removed from the sample obtained in step S200, from which inorganic matter has been removed.

Due to their high polarity, asphaltenes can be removed using organic solvents that are non-polar or have very low polarity. Preferably, the polarity index of the organic solvent may be 0.5 or less, and more preferably 0. Examples of suitable solvents include heptane, hexane, and pentane, which have a polarity index of 0.

**In** particular, when pentane is mixed with the sample, the asphaltenes become insoluble and precipitate or coagulate, which facilitates their separation and removal.

Step (d): Classifying the sample obtained in step S300 into a group containing saturated hydrocarbons, a group containing aromatic hydrocarbons, and a group containing NSO compounds (S400).

**In** this step, the sample from which asphaltenes have been removed in step S300 is classified into a saturated hydrocarbon group, an aromatic hydrocarbon group, and an NSO compound group.

**In** the present disclosure, saturated hydrocarbons refer to compounds in which all covalent bonds between carbon-carbon or carbon-hydrogen atoms are single bonds, such as alkanes or paraffinic compounds, and particularly include cycloalkane-type hydrocarbons.

Aromatic hydrocarbons refer to compounds that contain stable, conjugated ring structures, such as benzene, with alternating double bonds. These include monocyclic arenes, fused aromatic ring systems, condensed aromatic hydrocarbons, and their substituted derivatives.

NSO compounds refer to organic compounds containing at least one selected from the group consisting of nitrogen (N), sulfur (S), or oxygen (O).

**In** particular, the classification may be carried out by sequentially separating:
a group containing saturated hydrocarbons using a first solvent,
a group containing aromatic hydrocarbons using a second solvent, and
a group containing NSO compounds using a third solvent.

The first solvent may be an organic solvent having a polarity index of 0 to 0.5; the second solvent may be an organic solvent having a polarity index of 1 to 3; and the third solvent may be an organic solvent having a polarity index of 4 to 5.

In particular, hexane may be used as the first solvent to isolate saturated hydrocarbons.

In addition, a mixture of hexane and dichloromethane (DCM) in a volume ratio of 8:2 to 4:6 (v/v) may be used as the second solvent to isolate aromatic hydrocarbons.

In addition, a mixture of chloroform and methanol in a volume ratio of 90:10 to 99:1 (v/v) may be used as the third solvent to isolate NSO compounds.

In addition, the flow rate of the first solvent may be 0.1 to 1 drop/sec, where the flow rate of the second solvent may be 0.5 to 1.5 drop/sec, and the flow rate of the third solvent may be 1.5 to 2.5 drop/sec (see Table 1).

By controlling the polarity through the selection and mixing ratio of solvents, and by adjusting the volume and flow rate of the solvents, the sample can be effectively classified into saturated hydrocarbons, aromatic hydrocarbons, and NSO compounds.

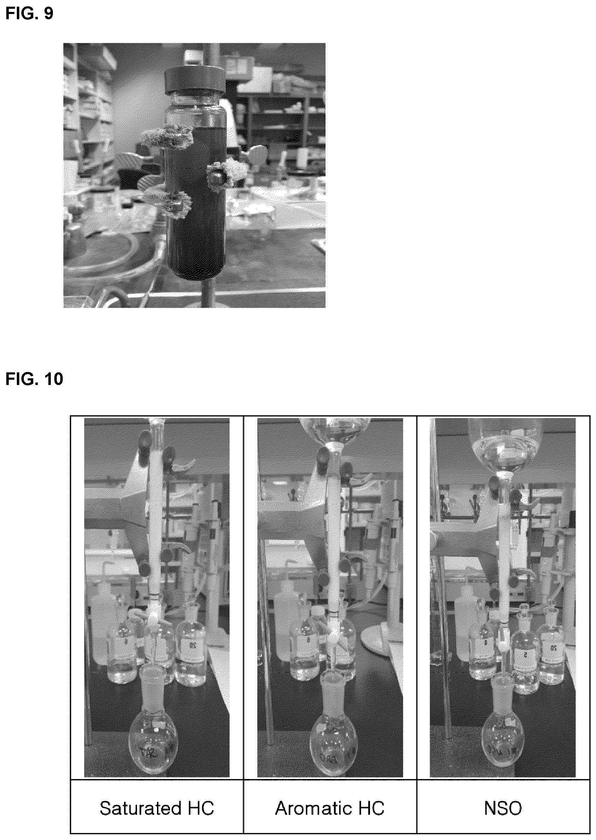

The classification may be performed using an alumina activation method. In particular, an alumina column may be used (see FIG. 10).

Step (e): Detecting components included in each of the samples classified in step S400 (S500).

This step may be performed using GC, GC/MS, or both GC and GC/MS. Biomarkers in the sample may be detected and identified based on the result graphs obtained from the GC and/or GC/MS analysis.

In particular, the biomarkers detected from the sample classified as the saturated hydrocarbon group may include Triterpanes, 25norHopanes, and Steranes.

The biomarkers detected from the sample classified as the aromatic hydrocarbon group may include Naphthalenes, Phenanthrenes, and Benzothiophenes.

In the sample classified as the NSO compound group, the detected biomarkers may include pyrrolidines and carboxylic acids.

### Exemplary embodiments

Hereinafter, the present disclosure will be described in more detail with reference to specific exemplary embodiments. However, the scope of the present disclosure is not limited to the following embodiments, and various modifications may be made without departing from the spirit of the present disclosure.

### 1. Sample Preparation

The prepared samples are finely ground using an agate mortar. Prior to use, the agate mortar is cleaned with methanol and dichloromethane (DCM) to completely remove any residual organic matter (see FIG. 2). Samples 1, 2, and 3 were all collected from a borehole drilled into the Devonian Grosmont Formation in Alberta, Canada.

### 2. Organic Matter Extraction

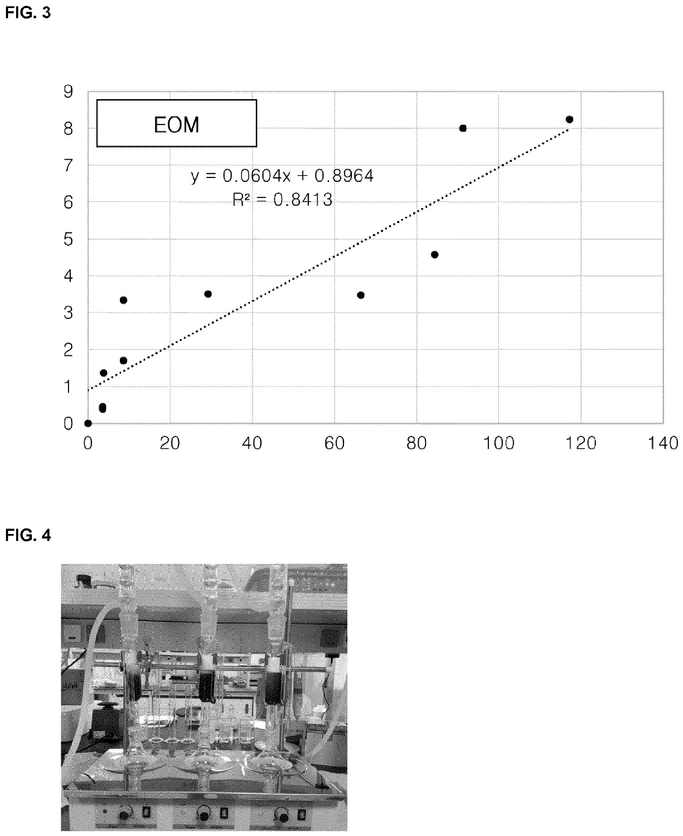

The amount of sample used for extraction was determined based on geochemical analysis results (see FIG. 3).

### (1) Samples containing rock and sediment

In the case of rock and sediment samples, organic matter was extracted using a Soxhlet apparatus (see FIG. 4).

The Soxhlet apparatus is a device in which an extraction chamber is mounted on a round-bottom (distillation) flask, and a condenser, through which cooling water flows, is placed above the chamber. When a cylindrical filter (thimble) is inserted into the extraction chamber and the solvent in the round-bottom flask is heated, the solvent vapor travels through the chamber, condenses in the condenser, and accumulates in the extraction chamber, thereby dissolving the soluble organic components in the sample.

Once the accumulated solvent reaches the top of the siphon tube by the siphon on the right side, the accumulated solvent is completely returned to the round-bottom flask, and fresh solvent begins to accumulate in the extraction chamber again. Through this repeated cycle, the sample is continuously exposed to fresh solvent, and the soluble organic matter is gradually dissolved and collected in the solvent flask. To extract organic matter that is soluble in organic solvents, a 1:1 (v/v) mixture of dichloromethane (DCM) and methanol (MeOH) is used as the extraction solvent.

Copper wire is added to the sample to remove free sulfate. Since free sulfate can affect chromatograms during subsequent GC or GC-MS analysis, it is desirable to remove it during the initial organic matter extraction process.

The reaction for removing free sulfate proceeds as follows:

Cu + H₂S ---> CuS (s) + H₂

Because the surface of the copper wire used for removing free sulfate is typically coated with copper oxide (CuO), the oxide layer must first be removed. In order to remove CuO, 10% hydrochloric acid (HCl) is used, as shown in the following reaction:

CuO + HCl ----> H₂O + CuCl

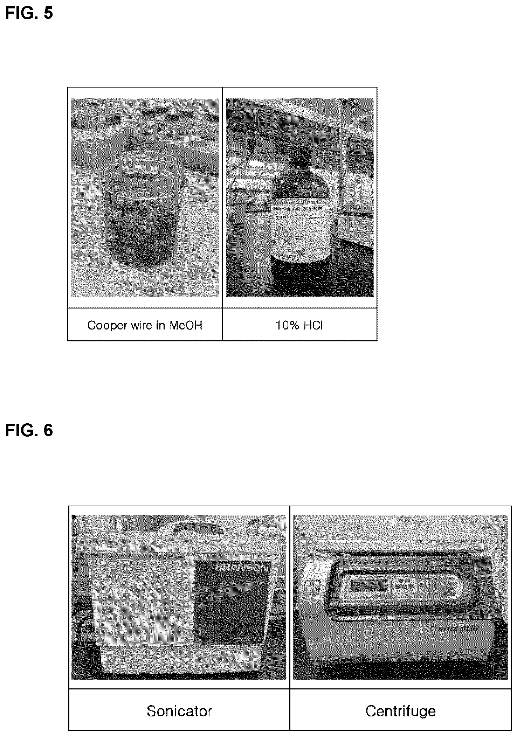

The copper wire is immersed in the HCl solution for approximately one minute, then neutralized using distilled water. It is then thoroughly rinsed with DCM and MeOH to remove any residual organic matter. The prepared copper wire is stored in MeOH to prevent exposure to air (see FIG. 5).

### (2) Samples containing oil and bitumen

In the case of oil and bitumen samples, which contain a high concentration of organic matter that is highly soluble in organic solvents, organic matter can be easily extracted using an ultrasonic sonicator (see FIG. 6). The solvent used is the same as that in the Soxhlet method. That is, a 1:1 (v/v) mixture of dichloromethane (DCM) and methanol (MeOH) is used as the solvent.

The sample is placed into a centrifuge tube, which is then filled with the solvent mixture. The tube is subjected to ultrasonic extraction for 30 minutes using the sonicator. To effectively separate the organic matter dissolved in the solvent from the remaining solids, the mixture is centrifuged at 1,000 rpm for 10 minutes. The supernatant organic solvent is then transferred to a round-bottom flask. This process is repeated three times.

### 3. Removal of Inorganic Matter

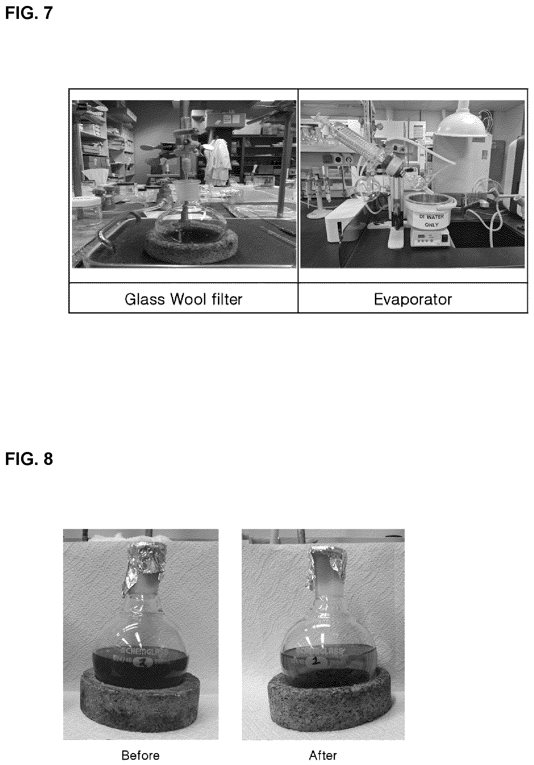

To remove clay minerals from the extracted organic matter, a filtration process is performed. In this process, glass wool is used as the filter medium. A three-layer filter is prepared using glass wool, and the solvent is passed through the filter. The filtered solvent is then completely distilled at approximately 37 °C using an evaporator (see FIG. 7).

The effectiveness of the filtration can be assessed based on the clarity of the filtered solvent. If the solvent appears transparent, the filtration is considered to have been effective (see FIG. 8).

### 4. Removal of Asphaltenes

Asphaltenes are highly polar and high-molecular-weight substances. Because of their strong interference in biomarker analysis, it is essential to remove them effectively.

Due to their high polarity, asphaltenes can be efficiently removed using a non-polar organic solvent such as pentane.

Pentane, with a polarity index of 0, is a completely non-polar organic solvent. When asphaltenes come into contact with pentane, precipitation occurs: Asphaltene + Pentane → Precipitation

After adding pentane to the sample to completely dissolve the sample, the mixture is stored at -21 °C for more than 12 hours in a freezer. Asphaltenes can then be observed to have precipitated and coagulated (see FIG. 9).

Extracted Organic Matter (EOM) + Pentane
→ (1) Soluble Fraction : Moltene
→ (2) Insoluble Fraction : Asphaltene

At this point, the soluble phase in pentane is referred to as Moltene, and the insoluble, coagulated material is classified as Asphaltene.

The upper soluble phase is transferred to a round-bottom flask, while the lower coagulated asphaltene fraction is dissolved in DCM and transferred to a separate flask.

### 5. Fractionation of Biomarker Components

Fractionation is the process of separating the Moltene, from which asphaltenes have been removed, into saturated hydrocarbons (Saturated HC), aromatic hydrocarbons (Aromatic HC), and NSO compounds (NSO compounds).

Based on differences in polarity among the three components, the separation proceeds in order of increasing polarity, beginning with the least polar saturated hydrocarbons and ending with the most polar NSO compounds (see FIG. 10). This separation is carried out using an alumina column.

The alumina column is prepared as follows:
A glass tube with a diameter of 1 cm is filled with hexane, and then 7.5 g of alumina is measured and packed into the tube. The optimal height of the packed alumina column is approximately 6.5 to 7 cm.

The solvents used to separate each component are listed in Table 1 below.

Approximately 35 mg of the concentrated Moltene is adsorbed onto the alumina column by elution with solvent (see Table 4).

First, 17 mL of hexane is introduced into the prepared alumina column to elute the saturated hydrocarbons.

Next, 50 mL of a 7:3 (v/v) mixture of hexane and dichloromethane (DCM) is introduced to elute the aromatic hydrocarbons.

Finally, 50 mL of a 98:2 (v/v) mixture of chloroform and methanol (MeOH) is used to elute the NSO compounds.

FIG. 10 shows the visual appearance of the component-wise elution results when fractionation is performed according to the above procedure.

**[Table 1]**

| Compound | Solvent | Volume | Flow Rate |
|---|---|---|---|
| Saturated Hydrocarbons | Hexane | 17 mL | 0.5 drop/sec |
| **(Saturated HC)** | | | |
| Aromatic Hydrocarbons | Hexane:DCM (7v:3v) | 50 mL | 1 drop/sec |
| **(Aromatic HC)** | | | |
| NSO Compounds | Chloroform:MeOH (98v:2v) | 50 mL | 2 drop/sec |
| **(NSO Compound)** | | | |

### 6. Detection

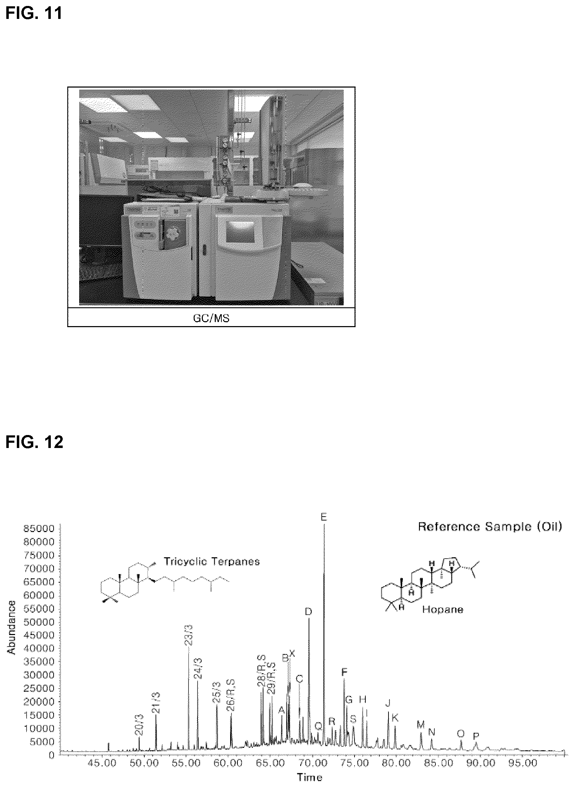

After separation, each component is analyzed using GC/MS (see FIG. 11). The injection concentration was set at approximately 3 mg/mL (see Table 5).

For GC/MS analysis, a Thermo system was used. The mass spectrometer (MS) was a quadrupole ion trap mass spectrometer. The DB-1 column was used in the same configuration as in the GC analysis.

The oven temperature program was as follows: initial temperature of 40 °C held for 1.5 minutes, then increased to 300 °C at a rate of 10 °C/min, followed by a hold at 300 °C for 34 minutes.

GC/MS analysis was performed in SIM (Selected Ion Monitoring) mode.

Here, the designated ion groups were as follows:
Saturated fraction: 66.0, 83.0, 85.0, 87.0, 110.0, 123.0, 124.0, 133.1, 134.1, 177.3, 183.3, 191.3, 205.3, 217.3, 218.3, 231.3, 232.3, 253.3 m/z
Aromatic fraction: 77.1, 99.1, 106.1, 119.1, 125.1, 128.1, 142.2, 156.2, 170.2, 178.2, 183.2, 184.2, 188.2, 192.2, 197.2, 198.2, 206.2, 211.2, 212.2, 216.3, 219.2, 219.3, 220.3, 228.3, 230.3, 231.3, 233.3, 234.3, 244.3, 245.3 m/z

The yield and concentration of the samples obtained at each step are presented in Tables 2 through 5.

**[Table 2]**

| **Sampl e No.** | EOM Extraction | | | | |
|---|---|---|---|---|---|
| | **Sample Weight** | **Container Weight** | **Container+ EOM Weight** | **Total EOM Weight** | EOM **Concentration s** |
| | **(g)** | **(g)** | **(g)** | **(mg)** | **(%)** |
| 1 | 10.2 | 14.05345 | 15.31778 | 1264.33 | 12.40 |
| 2 | 10.3 | 13.96393 | 15.29676 | 1332.83 | 12.94 |
| 3 | 10.6 | 14.06162 | 14.32158 | 259.96 | 2.45 |

**[Table 3]**

| **N o .** | **Asphaltene Removal** | | | | | | | | |
|---|---|---|---|---|---|---|---|---|---|
| | **EOM Weight** | **Asphaltene Container Weight** | **Container** + **Asphaltene Weight** | **Total Asphaltene Weight** | **Asphaltene /EOM** | **Moltene Container Weight** | **Container** + **Moltene Weight** | **Total Moltene Weight** | **Moltene /EOM** |
| | **(mg)** | **(g)** | **(g)** | **(mg)** | **(%)** | **(g)** | **(g)** | **(mg)** | **(%)** |
| 1 | 95.17 | 3.92482 | 3.94613 | 21.31 | 22.39 | 3.91784 | 4.00037 | 82.53 | 86.72 |
| 2 | 111.87 | 3.91199 | 3.92324 | 11.25 | 10.06 | 3.87191 | 3.98631 | 114.4 | 102.26 |
| 3 | 88.58 | 3.91679 | 3.93738 | 20.59 | 23.24 | 3.94345 | 4.02495 | 81.5 | 92.01 |

**[Table 4]**

| **N o .** | **Classification** | | | | | | | | | | | | | |
|---|---|---|---|---|---|---|---|---|---|---|---|---|---|---|
| | **Molte ne Weigh t** | **SAT Contain er Weight** | **Contai ner +SAT Weight** | **Total SAT Weig ht** | **SAT /EOM Conc entra tion** | **ARO Contai ner Weight** | **Contain er +ARO Weight** | **Total ARO Wei ght** | **ARO /EOM Conc entrat ion** | **NSO Contai ner Weight** | **Contai ner +NSO Weight** | **Total NSO Wei ght** | **NSO /EOM Conc entra tion** | **Reco very Rate** |
| | **(mg)** | **(g)** | **(g)** | **(mg)** | **(%)** | **(g)** | **(g)** | **(mg)** | **(%)** | **(g)** | **(g)** | **(mg)** | **(%)** | **(%)** |
| 1 | 35.44 | 3.93626 | 3.94575 | 9.49 | 26.78 | 3.87509 | 3.88327 | 8.18 | 23.08 | 3.85339 | 3.86558 | 12.19 | 34.40 | 84.26 |
| 2 | 35.96 | 3.94182 | 3.95284 | 11.02 | 30.65 | 3.93069 | 3.94063 | 9.94 | 27.64 | 3.85166 | 3.85974 | 8.08 | 22.47 | 80.76 |
| 3 | 35.69 | 3.91702 | 3.92734 | 10.32 | 28.92 | 3.92936 | 3.94039 | 11.03 | 30.91 | 3.89319 | 3.90400 | 10.81 | 30.29 | 90.11 |

**[Table 5]**

| **Sample No.** | **Injection Concentrations for GC, GC-MS Analysis** | | | | | |
|---|---|---|---|---|---|---|
| | **SAT Weight** | **Hexane Volume** | **SAT Injection Concentration** | **ARO Weight** | **Hexane Volume** | **ARO Injection Concentration** |
| | **(mg)** | **(mL)** | **(mg/mL)** | **(mg)** | **(mL)** | **(mg/ml)** |
| 1 | 9.49 | 3 | 3.2 | 8.18 | 2.6 | 3.1 |
| 2 | 11.02 | 3.6 | 3.1 | 9.94 | 3.3 | 3.0 |
| 3 | 10.32 | 3.5 | 2.9 | 11.03 | 3.7 | 3.0 |

### 7. Analysis

### (1) Saturated Hydrocarbons

### (1-1) Triterpanes (m/z 191)

Triterpanes are compounds typically found in oil or bitumen and are derived from triterpenoids synthesized by microorganisms. They can be identified in the m/z 191 fragmentograms.

Detected compounds include C₁₉-C₂₅ tricyclic terpanes, C₂₈-C₃₅ ab hopanes, and Gammacerane (see FIG. 12 and Table 6).

### (1-2) 25norHopane (Demethylated Hopane) (m/z 177)

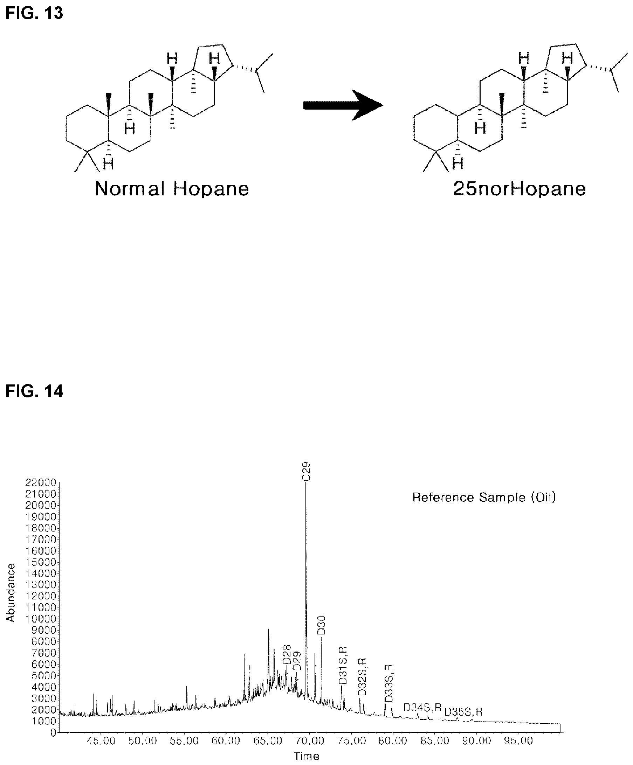

25norHopane is an organic compound derived from Hopanes through demethylation at the C-25 position (see FIG. 13).

This can be identified in the 177 fragmentograms (see FIG. 14 and Table 6).

**[Table 6]**

| No. | Identify | Name | Chemical Formula |
|---|---|---|---|
| | 20/3 | Tricyclicterpane | C₂₀H₃₆ |
| | 21/3 | Tricyclicterpane | C₂₁H₃₈ |
| | 22/3 | Tricyclicterpane | C₂₂H₄₀ |
| | 23/3 | Tricyclicterpane | C₂₃H₄₂ |
| | 24/3 | Tricyclicterpane | C₂₄H₄₄ |
| | 25/3 | Tricyclicterpane(17R,17S) | C₂₅H₆₆ |
| | 26/3 | Tricyclicterpane(17R,17S) | C₂₆H₄₈ |
| | 28/3 | Tricyclicterpane(17R,17S) | C₂₈H₅₀ |
| | 29/3 | Tricyclicterpane(17R,17S) | C₂₉H₅₂ |
| a | 27Ts | 18a trisnorneohopane(Tₛ) | C₂₇H₄₄ |
| b | 27Tm | 17a trisnorhopane(Tₘ) | C₂₇H₄₆ |
| x | 25nor28ab | norhopane | C₂₇H₄₆ |
| c | 28ab | Bisnorhopane | C₂₈H₄₈ |
| y | 25nor30ab | norhopane | C₂₉H₅₀ |
| d | 29ab | ab norhopane | C₂₉H₅₀ |
| q | 29ba | ba norhopane | C₂₉H₅₀ |
| e | 30ab | ab hopane | C₃₀H₅₂ |
| r | 30ba | ba hopane | C₃₀H₅₂ |
| f | 31abR | 22Rab homohopane | C₃₁H₅₄ |
| g | 31ba | ba homohopane | C₃₁H₅₄ |
| s | 30G | gammacerane | C₃₀H₅₂ |
| h | 32abS | 22Sab bishomohopane | C₃₂H₅₆ |
| i | 32abR | 22Rab bishomohopane | C₃₂H₅₆ |
| j | 33abS | 22Sab trishomohopane | C₃₃H₅₆ |
| k | 33abR | 22Rab trishomohopane | C₃₃H₅₈ |
| m | 34abS | 22Sab tetrakishomohopane | C₃₄H₆₀ |
| n | 34abR | 22Rab tetrakishomohopane | C₃₄H₆₀ |
| o | 35abS | 22Sab pentakishomohopane | C₃₅H₆₂ |
| p | 35abR | 22Rab pentakishomohopane | C₃₅H₆₂ |

### (1-3) Steranes (m/z 217)

Steranes are tetracyclic saturated hydrocarbons composed of one five-membered ring and three six-membered rings. These compounds are predominantly found in higher organisms rather than cyanobacteria.

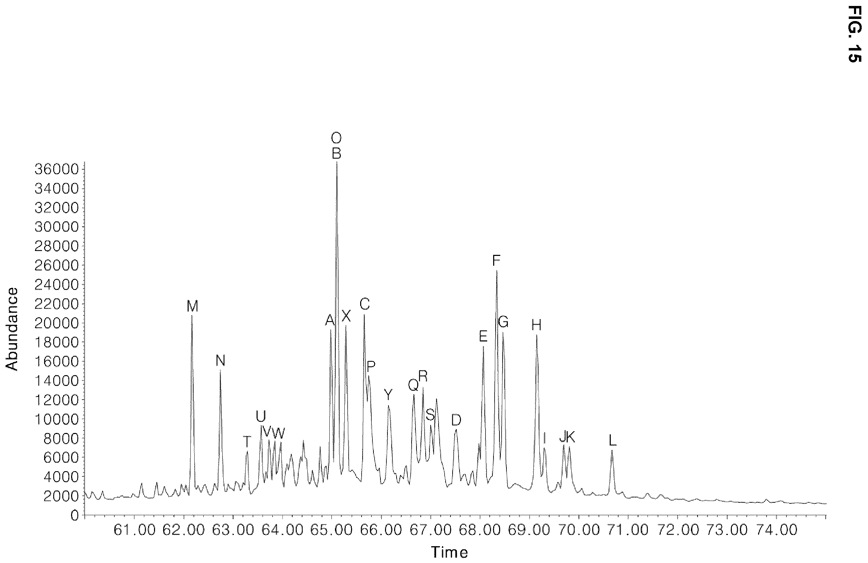

Sterane compounds can be identified in the m/z 217 fragmentograms (see FIG. 15 and Table 7).

**[Table 7]**

| No. | Identify | Name | Chemical Formula |
|---|---|---|---|
| | 21a | 5a sterane | C₂₁H₃₆ |
| | 22a | 5a sterane | C₂₂H₃₈ |
| m | 27dbS | 20Sba diacholestane | C₂₇H₄₈ |
| n | 27dbR | 20Rba diacholestane | C₂₇H₄₈ |
| t | 27daS | 20Sab diacholestane | C₂₇H₄₈ |
| u | 27daR | 20Rab diacholestane | C₂₇H₄₈ |
| v | 28dbS | 20Sba24-methyl-diacholestane | C₂₈H₅₀ |
| w | 28dbR | 20Rba24-methyl-diacholestane | C₂₈H₅₀ |
| a | 28daR | 20Rab24-methyl-diacholestane | C₂₈H₅₀ |
| a | 27aaS | 20Saaa cholestane | C₂₇H₄₈ |
| o | 29dbS | 20Sba24-ethyl-diacholestane | C₂₉H₅₂ |
| b | 27bbR* | 20Rabb cholestane | C₂₇H₄₈ |
| x | 27bbS* | 20Sabb cholestane | C₂₇H₄₈ |
| x | 28daS | 20Sab24-methyl-diacholestane | C₂₈H₅₀ |
| c | 27aaR | 20Raaa cholestane | C₂₇H₄₈ |
| p | 29dbR | 20Rba24-ethyl-diacholestane | C₂₉H₅₂ |
| y | 29daR | 20Rab24-ethyl-diacholestane | C₂₉H₅₂ |
| q | 28aaS | 20Saaa24-methyl-cholestane | C₂₈H₅₀ |
| r | 28bbR* | 20Rabb24-methyl-cholestane | C₂₈H₅₀ |
| r | 29daS | +20Sab24-ethyl-diacholestane | C₂₉H₅₂ |
| s | 28bbS* | 20Sabb24-methyl-cholestane | C₂₈H₅₀ |
| d | 28aaR | 20Raaa24-methyl-cholestane | C₂₈H₅₀ |
| e | 29aaS | 20Saaa24-ethyl-cholestane | C₂₉H₅₂ |
| f | 29bbR* | 20Rabb24-ethyl-cholestane | C₂₉H₅₂ |
| g | 29bbS* | 20Sabb24-ethyl-cholestane | C₂₉H₅₂ |
| h | 29aaR | 20Raaa24-ethyl-cholestane | C₂₉H₅₂ |
| k | 30bbS* | 20Sabb24-propyl-cholestane | C₃₀H₅₄ |
| l | 30aaR | 20Raaa24-propyl-cholestane | C₃₀H₅₄ |

### (2) Aromatic Hydrocarbons

### (2-1) Naphthalenes (m/z 128, 142, 156, 170, 184)

Naphthalenes are the simplest form of polycyclic aromatic hydrocarbons.

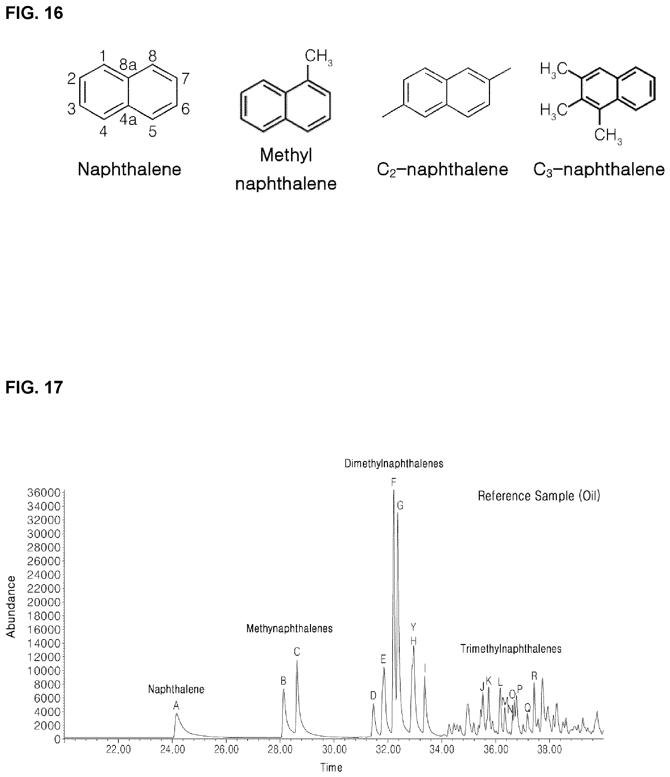

The structure of naphthalene consists of a pair of fused benzene rings (see FIG. 16).

Generally, Naphthalene is detected at m/z 128, while Methyl naphthalene sis detected at m/z 142.

In addition, C2-naphthalenes are detected at m/z 156, C3-naphthalenes are detected at m/z 170, and C4-naphthalenes are detected at m/z 184 (see FIG. 17).

### (2-2) Phenanthrene (m/z 178, 192, 206)

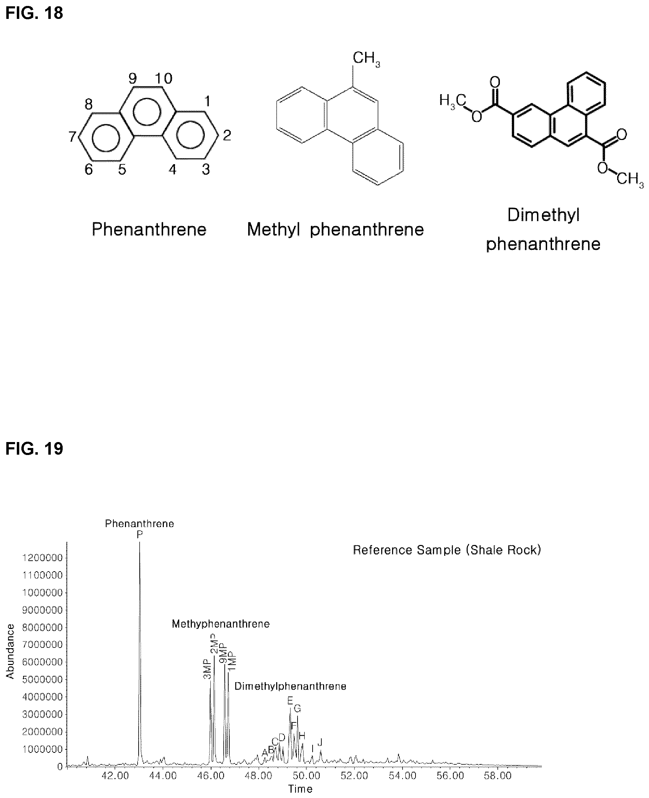

Phenanthrene is a polycyclic aromatic hydrocarbon composed of three benzene rings (see FIG. 18).

In general, Phenanthrene is detected at m/z 178, Methyl phenanthrene is detected at m/z 192, and Dimethyl phenanthrene is detected at m/z 206 fragment ion (see FIG. 19).

### (2-3) Benzothiophenes (m/z 184, 198, 212)

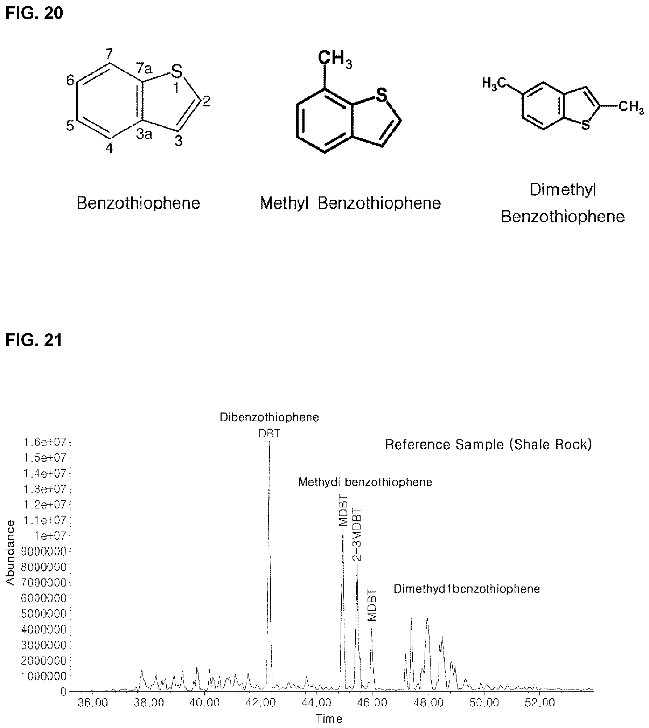

Benzothiophenes are sulfur-containing aromatic compounds with the molecular formula C₈H₆S, and have a structure similar to that of naphthalene (see FIG. 20).

Benzothiophenes are commonly found in petroleum-related depositional environments.

In the above, exemplary embodiments of the method for separating and analyzing biomarkers from sediments according to an exemplary embodiment of the present disclosure have been described. Moreover, it will be appreciated that various modifications to these exemplary embodiments are possible without departing from the scope of the present disclosure.

The scope of the present disclosure should therefore not be limited to those exemplary embodiments described above, but should be defined by the following claims and their equivalents.

In other words, the foregoing exemplary embodiments are to be understood as illustrative rather than restrictive in all respects, and the scope of the present disclosure is indicated by the following claims rather than the detailed description. All modifications or variations derived from the meaning, scope, and equivalent concepts of the claims should be interpreted as being included within the scope of the present disclosure.

## Claims

1. A method for separating and analyzing biomarkers, comprising:
(a) extracting organic matter from an original sample;
(b) removing inorganic matter from a sample obtained in step (a);
(c) removing asphaltene from a sample obtained in step (b);
(d) classifying a sample obtained in step (c) into a group containing saturated hydrocarbons, a group containing aromatic hydrocarbons, and a group containing NSO compounds; and
(e) detecting components included in each of the group containing saturated hydrocarbons, the group containing aromatic hydrocarbons, and the group containing NSO compounds, all of which are obtained in step (d).

2. The method of claim 1,
wherein the sample in step (a) includes a mixture of one or more selected from the group consisting of soil, rock, sediment, marine sediment, sedimentary rock, source rock, and reservoir rock.

3. The method of claim 1 or 2,
wherein step (a) includes grinding the original sample prior to the extraction of organic matter.

4. The method of any one of claims 1 to 3,
wherein step (a) includes performing distillation with an organic solvent on a sample including rock, sediment, or a mixture thereof, and
wherein the sample does not contain oil or bitumen.

5. The method of any one of claims 1 to 4,
wherein step (a) includes performing ultrasonic crushing after adding an organic solvent to a sample containing oil, bitumen, or a mixture thereof.

6. The method of any one of claims 1 to 5,
wherein step (b) is performed using filtration.

7. The method of any one of claims 1 to 6,
wherein step (c) is performed by mixing a non-polar organic solvent with the sample obtained in step (b).

8. The method of any one of claims 1 to 7,
wherein step (d) is performed by sequentially separating:
a group containing saturated hydrocarbons using a first solvent,
a group containing aromatic hydrocarbons using a second solvent, and
a group containing NSO compounds using a third solvent.

9. The method of claim 8,
wherein the first solvent is an organic solvent having a polarity index of 0 to 0.5, the second solvent is an organic solvent having a polarity index of 1 to 3, and the third solvent is an organic solvent having a polarity index of 4 to 5.

10. The method of any one of claims 1 to 9,
wherein step (e) is performed using GC or GC/MS.
